# EUROPEAN PATENT APPLICATION

(11) **EP 1 489 163 A1**
(43) Date of publication of application: **22.12.2004**
(21) Application number: 03715540.5
(22) Date of filing: 27.03.2003
(51) Int. Cl.: C12N 5/02, C12N 5/06, C12N 5/08, A61K 35/12, A61K 38/19, A61P 25/00

(54) **METHOD OF INDUCING GROWTH OF NERVE STEM CELLS**

(30) Priority: 27.03.2002 JP 2002089624
(71) Applicant: Institute of Gene and Brain Science, Tokyo 160-0015 (JP)
(72) Inventor: TODA, Masahiro, Shinjuku-ku, Tokyo 160-8582 (JP); OKANO, Hideyuki, Shinjuku-ku, Tokyo 160-8582 (JP); KAWAKAMI, Yutaka, Shinjuku-ku, Tokyo 160-8582 (JP); TOYAMA, Yoshiaki, Shinjuku-ku, Tokyo 160-8582 (JP); MIKAMI, Yuji, Shinjuku-ku, Tokyo 160-8582 (JP); SAKAGUCHI, Masanori, Shinjuku-ku, Tokyo 160-8582 (JP)
(74) Representative: Jones, Helen Marjorie Meredith
(86) International application number: PCT/JP2003/003868
(87) International publication number: WO 2003/080818

(57) **Abstract**

Method are provided for efficiently inducing, *in vivo*/*in vitro*, proliferation of neural stem cells, the most important for transplantation therapy etc. of nerve injuries or nerve function insufficiency. Also provided are methods for using the neural stem cells obtained by such a proliferation-inducing method. Mammalian nervous tissues containing neural stem cells are isolated, the neural stem cells are cultured in a culture medium containing growth factors, such as EGF and FGF, and then the neural stem cells are co-cultured with dendritic cells, such as an immature dendritic cell subset having the CD11c surface marker on the cell surface and blood cells, such as spleen cells and CD8-positive T cells. Or the neural stem cells after culture are cultured in the presence of GM-CSF. Alternatively, the neural stem cells after culture are cultured in the culture supernatant of dendritic cells and/or blood cells.

## Description

### TECHNICAL FIELD

The present invention relates to methods for inducing proliferation of neural stem cells, which are undifferentiated neural cells with pluripotency, use of neural stem cells obtained by such proliferation-inducing methods, proliferation-inducing sets of neural stem cells, and use of such proliferation-inducing sets of neural stem cells.

### BACKGROUND ART

Many spinal cord injuries are traumatic and they are caused by traffic accidents, sport accidents, industrial accidents and the like. Non-traumatic ones are attributed to inflammation, bleeding, tumors, spine deformation and the like. The pathologic conditions include crush and pressure lesions of the spinal cord based on bleeding and edema in the spinal substance, causing neuropathy corresponding to the injured site. As main clinical manifestations, paresi or paralysis and anesthesia occur below the injury level. In the case of cervical cord injuries, respiratory paralysis and hyperthermia (or hypothermia) are observed as the characteristic complications. The improvement of the above-mentioned neuropathy, especially the amelioration of dyskinesia is directly linked to prevention of the increasing number of the bedridden elderlies or to enhancement of their quality of life (QOL); it is becoming more important with the increasing average life span in recent years.

Treatment for the above-mentioned spinal cord injuries include surgical operations for eliminating physical compression and injury and steroid therapy for spine edema in the acute phase after injury (N.Engl J. Med. 322, 1405-1411, 1990, J.Neurosurg 93, 1-7, 2000). It is reported that among steroids, high-dose administration of methylprednisolone is effective in improving neurological symptoms associated with spinal cord injuries (J.Spinal Disord. 5(1), 125-131, 1992). However, excessive administration of steroids is also accompanied by strong systemic side effects and difficult to control. In addition, in the case of the spinal cord injuries accompanied by an infection, such administration causes a problem of reducing the function of defending against infection. Furthermore, currently, the validity of high-dose steroid therapy itself has become controversial. As described above, to date, there have been no effective therapeutic agents for spinal cord injuries; the development of a new therapeutic agent is desired.

There are other reports on a therapeutic method for spinal cord injuries: the method for transplanting a therapeutically effective dose of astrocytes pretreated by an inflammation-related cytokine in vitro to the injured site in the central nervous system (CNS)(National Publication of International Patent Application No. 2000-503983); and the method for promoting neurocladism in the mammalian CNS by administering monocular phagocytes (monocytes, macrophages, etc.) of the same kind to the injured or disease site, or to the CNS in the vicinity of such sites (J. Mol. Med.77, 713-717, 1999, andN.Neurosci. 19 (5) 1708-16, 1999, Neurosurgery 44 (5) , 1041-5, 1999, Trends. Neurosci 22 (7), 295-9, 1999, National Publication of International Patent Application No. 1999-13370, etc.). In addition, there is another report that the recovery of movement maintenance after a spinal cord injury was promoted by vaccination with spinal cord homogenate or administration of T cells specific to the myelin basic protein, which is a myelin sheath protein, although a clear mechanism of the action is not known (Neuron 24, 639-647, 1999, Lancet 354,286-287, 2000).

Recently in Europe and America, a clinical trial has been carried out in which embryonic brain cells were transplanted into patients with Parkinson's disease, in which dopaminergic neurons in the mesencephalic substantia nigra are degenerated and lost (Piccini P., et al., Nat Neurosci., 2, 1999, Freed C.R., et al., N.Engl.J.Med., 344, 2001). It was revealed that this treatment method improves the movement ability of patients under the age of 60. It is thought that to perform this transplantation therapy on one patient with Parkinson's disease, as many as 5 to 10 aborted embryos are required.

On the other hand, a selective culture method of neural stem cells called the neurosphere method was developed by the group of Weiss et al. in 1992, whereby a turning-point was marked in the history of studies of neural stem cells (Reynolds B.A, et al.J.Neurosci., 12, 1992). In this method, cell colonies containing neural stem cells are cultured in a serum-free liquid medium containing some growth factor(s). Here, only neural stem cells proliferate and suspend as cell aggregates (neurospheres). Furthermore, when the neurospheres that have generated are disaggregated into individual cells and cultured in the aforementioned serum-free liquid medium again, neurospheres are formed similarly. When these neurospheres are cultured in the aforementioned serum-free liquid medium without the growth factor(s), cell differentiation is induced so that three kinds of cells, neurons, astrocytes, and oligodendrocytes are formed.

Meanwhile, dendritic cells (DCs) are a population of cells with the arborescent form, derived from hematopoietic stem cells, and are distributed widely in the living body. Immature dendritic cells recognize and incorporate foreign substances, including viruses and bacteria that have invaded individual tissues, digest and degrade them to generate peptides in the process of moving to the T cell region of the lymphoid organs, and bind the peptides to MHC molecules and present them on the cell surface, thereby bearing a role of antigen-presenting cells that activate antigen-specific T cells and induce immunological responses (Ann.Rev.Immunol.9, 271-296, 1991, J.Exp.Med.185, 2133-2141, 1997).

Although dendritic cells are distributed widely, their density in individual tissues was not high; it was difficult to prepare dendritic cells in large numbers. However, addition of some differentiation/growth factor(s) to the culture of immature precursors has enabled easy *in vitro* preparation of a multitude of dendritic cells. Based on that, use of dendritic cells as an immunopotentiator has started to be discussed (J. Exp. Med. 183, 7- 11, 1996). For example, in a feeble tumor immunological response, immunological responses are specifically enhanced by pulsing dendritic cells with antigens. Animal experiments have shown that dendritic cells that have presented a tumor-derived protein or antigen peptide induce specific CD8 + cytotoxic T cells. It is reported that likewise in humans some tumors are reduced or lost by returning a tumor-derived protein or antigen peptide to the living body together with dendritic cells.

Neural stem cells are undifferentiated neural cells having capability, i.e., pluripotency, to differentiate into three kinds of cells constituting the central nervous system, called neurons, astrocytes, and oligodendrocytes, together with a self-replication ability to divide and proliferate (Temple S., Nature, 414, 2001). In recent years, it has been found that neural stem cells are present even in the adult brain with extremely low regenerative capacity. Further, isolation and preparation of human neural stem cells have become possible. Thus neural stem cells have now become the focus of special attention in the current regenerative medicine research.

Recently, a method has been developed for inducing differentiation of stem cells that have proliferated *in vitro* into dopaminergic neurons. If transplantation of cells induced by this method as donor cells becomes possible, that is expected to be a therapeutic method for Parkinson's disease far more superior to the current method requiring a large number of aborted embryos. In addition, it is expected that such therapy as transplantion of cells prepared in large quantities by getting stem cell biology into full use will be performed on various neurological diseases from now on. An object of the present invention is to provide methods, which are the most important for such transplantation therapy, for efficiently inducing proliferation of neural stem cells *in vitro* and in other manners, and others.

### DISCLOSURE OF THE INVENTION

It is considered that in the adult spinal cord injuries, while endogenous neural stem cells are present within the spinal cord, de novo neurogenesis is suppressed and proliferation of astrocytes alone occurs. Since simple introduction of neural stem cells into an injured site will result in glial formation, without neuronal formation, such treatment is unlikely to lead to improvement of the pathologic condition. Therefore, in addition to neural stem cell transplantation, arrangement of the microenvironment for producing neurons is essential. On the other hand, an antigen-specific immunological reaction mainly involving T cells is present as one of the host defense mechanisms. The central nervous system is in the unique environment completely isolated from the immune system because of the presence of the blood brain barrier, extremely low expression of MHC antigens, lack of the lymphatic tissue, and so forth. Thus, based on the assumption that the immune system eliminates and restores the diseased tissue, the present inventors attempted introduction of the immune system to the injured nervous tissue. Specifically, the inventors found that proliferation of endogenous neural stem cells are induced by administering, to injured nervous tissue, dendritic cells, the most important cells for regulating the immune system, and/or granulocyte-macrophage colony stimulating factor (GM-CSF) , a cytokine important for induction and proliferation of dendritic cells. The inventors also found that in vitro proliferation of neural stem cell is induced by co-culture with dendritic cells. The present invention has thus been accomplished.

The present invention relates to a method for inducing proliferation of a neural stem cell, including contacting the neural stem cell with at least one of a dendritic cell, a blood cell, the culture supernatant of the cell, or granulocyte-macrophage colony stimulating factor (GM-CSF) (claim 1); the method for inducing proliferation of a neural stem cell of claim 1, including contacting the neural stem cell with at least one of a dendritic cell, a blood cell, and granulocyte-macrophage colony stimulating factor (GM-CSF) in a culture medium (claim 2); the method for inducing proliferation of a neural stem cell of claim 2, including isolating a mammalian nervous tissue containing the neural stem selectively culturing the neural stem cell in a culture medium containing a growth factor, and then co-culturing the neural stem cell with a dendritic cell and/or a blood cell (claim 3); the method for inducing proliferation of a neural stem cell of claim 2, including isolating a mammalian nervous tissue containing the neural stem cell, selectively culturing the neural stem cell in a culture medium containing a growth factor, and then culturing the neural stem cell in the culture supernatant of a dendritic cell and/or a blood cell (claim 4) ; the method for inducing proliferation of a neural stem cell of any one of claims 2 to 4, in which the culture medium containing the growth factor is a culture medium containing at least EGF and/or FGF (claim 5); the method for inducing proliferation of a neural stem cell of any one of claims 1 to 5, in which the dendritic cell are an immature dendritic cell subset having the CD11c surface marker on the cell surface or a mature dendritic cell subset derived from the immature dendritic cell subset (claim 6) ; and the method for inducing proliferation of a neural stem cell of any one of claims 1 to 6, in which the blood cell is a spleen cell, a T cell, a monocyte, a neutrophil, an eosinophil, or a basophil (claim 7).

The present invention also relates to a set for inducing proliferation of a neural stem cell, including at least one of a dendritic cell, a blood cell or the culture supernatant of the cell, or the granulocyte-macrophage colony stimulating factor (GM-CSF) (claim 8); the set for inducing proliferation of a neural stem cell of claim 8, further including a culture medium containing a growth factor (claim9); and the set for inducing proliferation of a neural stem cell of claim 9, in which the culture medium containing the growth factor is a culture medium containing at least EGF and/or FGF (claim 10) ; the set for inducing proliferation of a neural stem cell of any one of claims 8 to 10, in which the dendritic cell is an immature dendritic cell subset having the CD11c surface marker on the cell surface or a mature dendritic cell subset derived from the immature dendritic cell subset (claim 11), the set for inducing proliferation of a neural stem cell of any one of claims 8 to 11, in which the blood cell is a spleen cell, a T cell, a monocyte, a neutrophil, an eosinophil, or a basophil (claim 12) .

The present invention also relates to a therapeutic agent for a nerve injury or nerve function insufficiency, containing as an active ingredient the neural stem cell obtained by the method for inducing proliferation of any one of claims 1 to 7 (claim 13); a therapeutic agent for a nerve injury or nerve function insufficiency, containing as an active ingredient a set for inducing proliferation of any one of claims 8 to 12 (claim 14); a therapeutic agent for cerebral infarction, including containing granulocyte-macrophage colony-stimulating factor (GM-CSF) as an active ingredient (claim 15); a therapeutic method for a nerve injury or nerve function insufficiency, including administering the neural stem cell obtained by the method for inducing proliferation of any one of claims 1 to 7 (claim 16); a therapeutic method for a nerve injury or nerve function insufficiency, including administering the set for inducing proliferation of any one of claims 8 to 12 (claim 17) ; and a therapeutic method for cerebral infarction, including administering granulocyte-macrophage and colony-stimulating factor (GM-CSF) (claim 18).

### BRIEF DESCRIPTION OF THE DRAWINGS

FIG. 1 shows the area setting for measurement of the number of Musashi-1-positive cells after transplantation of dendritic cells.
FIG. 2 shows the results of immunostaining using anti-Musashi-1 antibody in the dendritic cell (DC)- and the RPMI1640 (RPMI) -transplanted groups, especially representative time-course tissue sections cranially from proximal injured region.
FIG. 3 shows time-course changes in the number of Musashi-1-positive cells in each of the regions in the dendritic cell- and the RPMI1640-transplanted groups.
FIG. 4 shows the results of analyses of nerve cells by transplantation of dendritic cells.
   A: Results (representative sections) of the immunostaining using anti-Hu antibody and anti-BrdU antibody in the dendritic cell-transplanted group on day 14 after injury.
   B: High-magnified images of the region in a square in A., which show the results of immunostaining with each of the antibodies. C: Serial cross-sectional images of cells shown in B.
   D: Images of time-course changes in the number of Hu-positive and BrdU-positive cells in each of the dendritic cell-transplanted group and the RPMI1640-transplanted group (control).
   E: Results (representative stainings) of the triple staining of the dendritic cell-transplanted group on day 14 after injury by TUNEL staining and double immunostaining using anti-Hu antibody and anti-BrdU antibody.

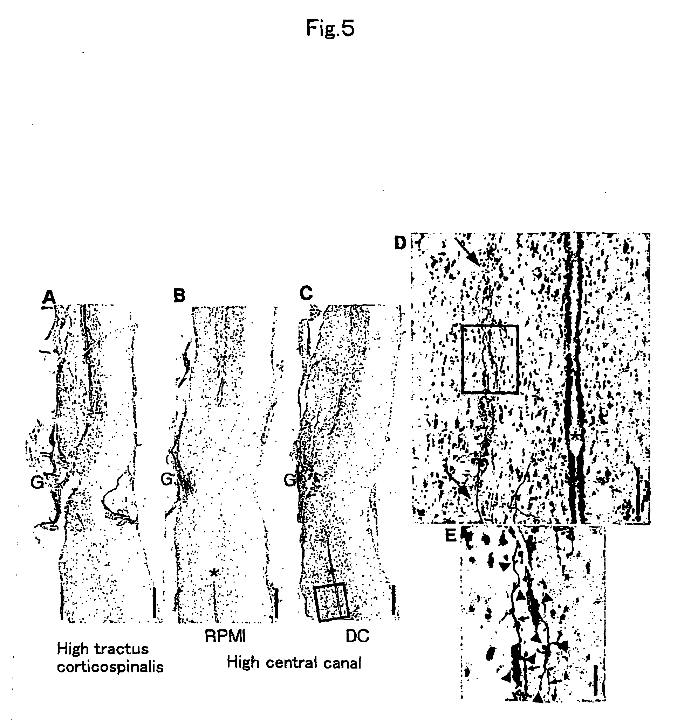

FIG. 5 shows the results of the effect of dendritic cell transplantation on cut nerve axons.
   A: A representative section indicating the anterograde tracer BDA-positive tractus corticospinalis in the dendritic cell-transplanted group in month 4 after injury. G in the figure shows gelfoam inserted into the injured region.
   B: A representative section indicating BAD-positive nerve axons in the periphery of the central canal in the RPMI1640-transplanted group (control) in month 4 after injury.
   C: A representative section indicating BAD-positive nerve axons in the periphery of the central canal in the dendritic cell-transplanted group in month 4 after injury.
   D: A high-magnified image of the region shown in a square in C.
   E: A high-magnified image of the region shown in a square in D.

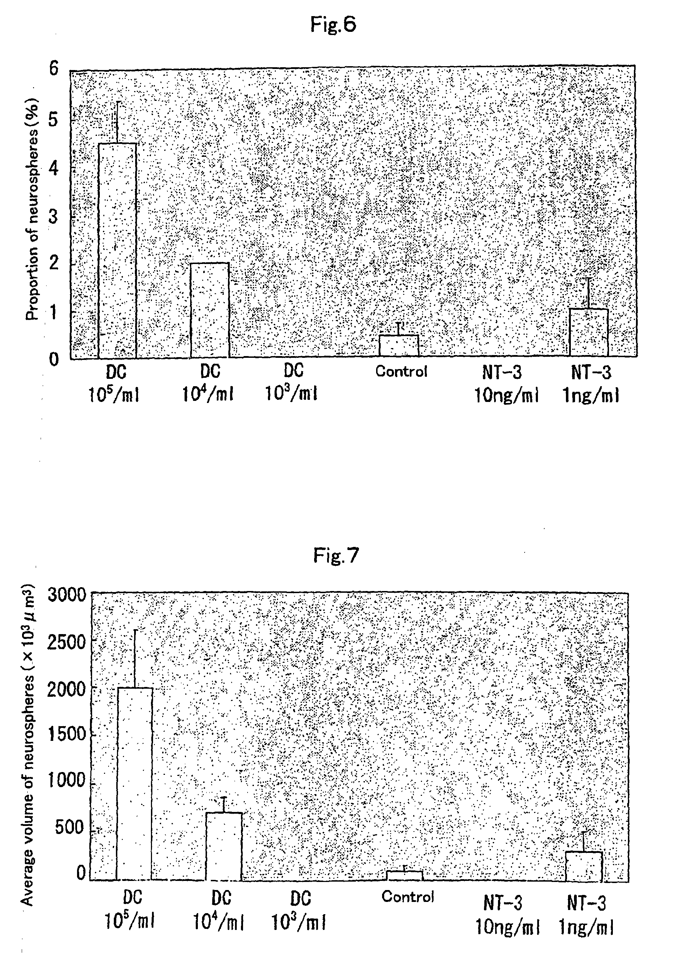

FIG. 6 shows the numbers of neurospheres formed by co-culture with dendritic cells, each indicating an average value of four wells analyzed.
FIG. 7 shows the volumes of the neurospheres formed by co-culture with dendritic cells, each indicating an average value of four wells analyzed.

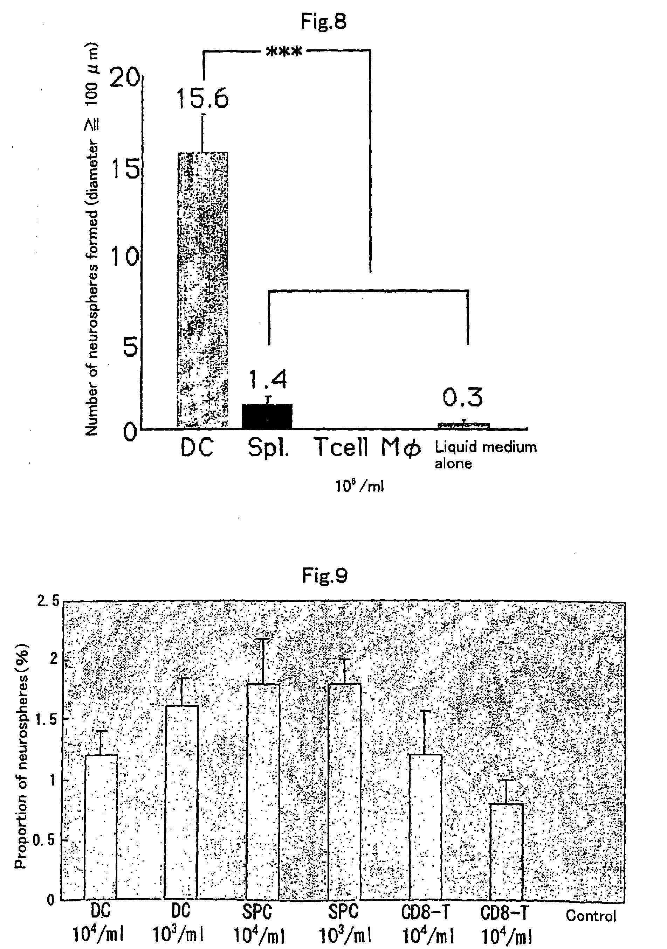

FIG. 8 shows the numbers of secondary neurospheres (with a diameter of 100 µm or more) formed by 7-day co-culture of 10⁵ immunocytes (10⁶/ml) of dendritic cells, spleen cells, T cells, and macrophages with 100 neural stem cells that have formed primary neurospheres.
FIG.9 shows the numbers of neurospheres formed by the culture using the culture supernatants of dendritic cells, spleen cells, and T cells, each indicating an average value of four wells analyzed.

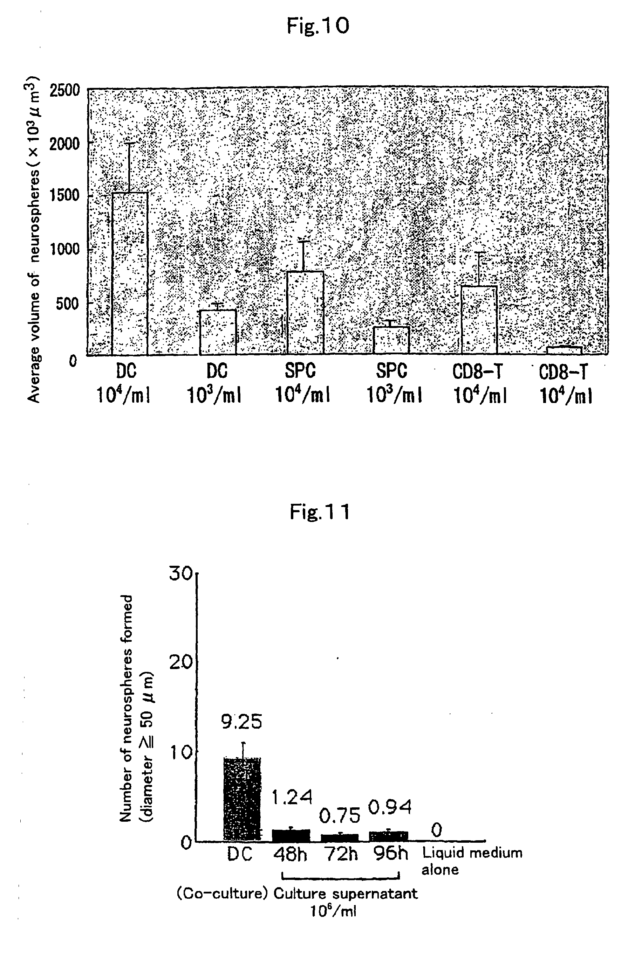

FIG. 10 shows the volumes of neurospheres formed by the culture using the culture supernatants of dendritic cells, spleen cells, and T cells, each representing an average value of neurospheres formed.
FIG. 11 shows the numbers of secondary neurospheres (with a diameter of 50 µm or more) formed by 7-day culture of 100 neural stem cells that have formed primary neurospheres, using the culture supernatant (10⁶/ml) of 10⁵ dendritic cells.

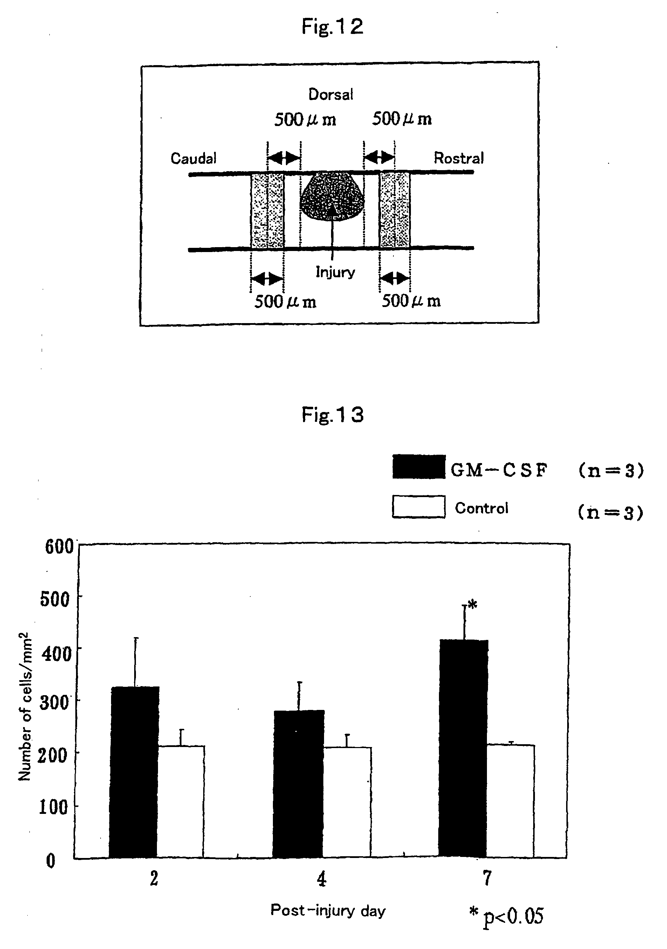

FIG. 12 shows the area setting for measurement of the number of Musashi-1 positive cells after administration of GM-CSF.
FIG. 13 shows the time-course changes in the number of Musashi-1 positive cells after administration of GM-CSF.

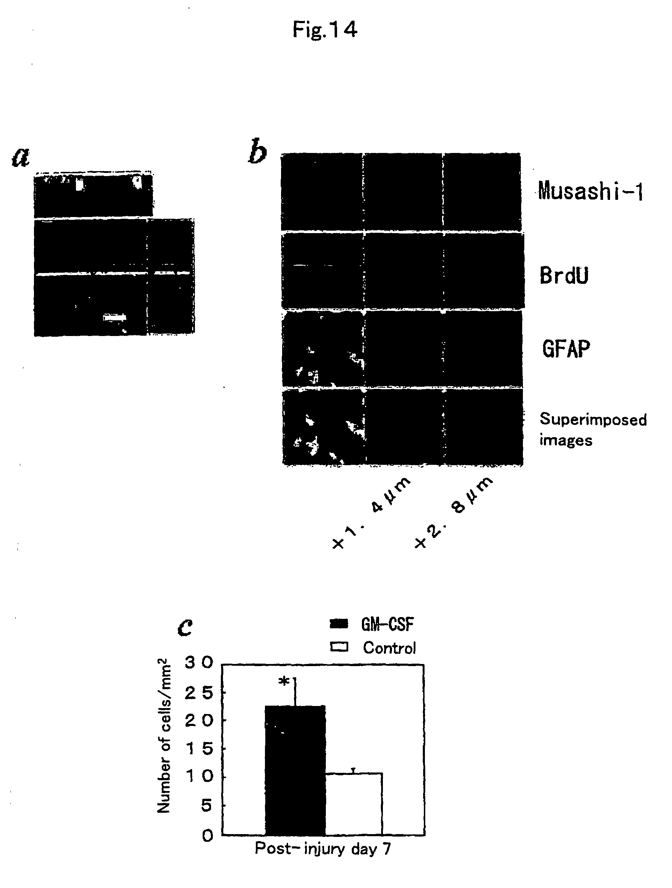

FIG. 14 shows the analyses of endogenous neural stem cells/precursors on day 7 after spinal cord injury.
   A: Staining images of representative sections triple-stained by Musashi-1 antibody, BrdU antibody, and GFAP antibody.
   B: Images analyzed by each antibody.
   C: Results of quantative measurements of the endogenous neural stem cells/precursors that are Musashi-1 (+) BrdU (+) and GFAP (-).

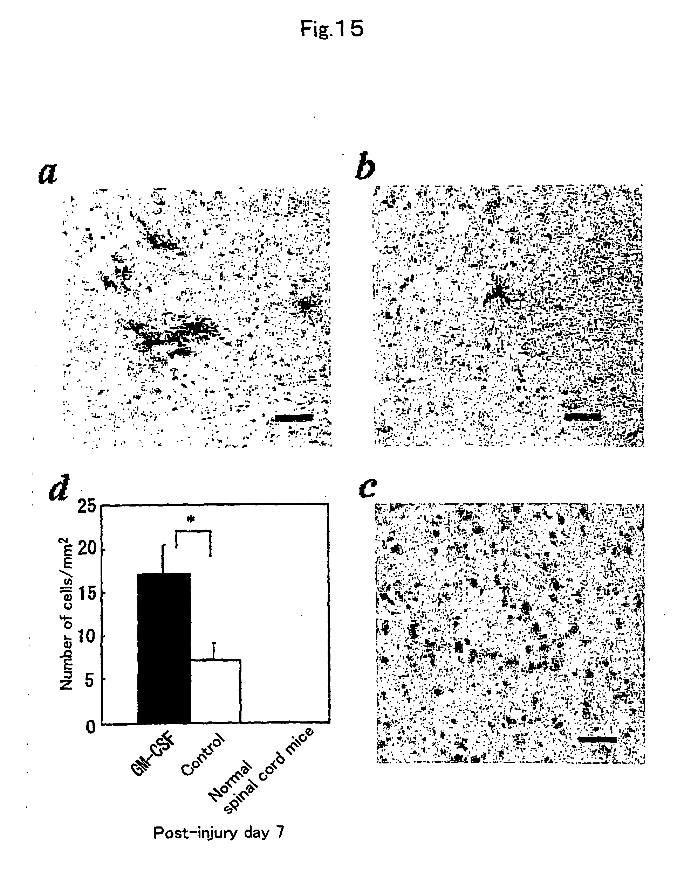

FIG. 15 shows the analyses of CD11c-positive dendritic cells on day 7 after spinal cord injury.
   a: The number of dendritic cells increases by the administration of GM-CSF to an injured site.
   b: CD11c-positive dendritic cells are observed in the injured spinal cord.
   c: Evident CD11c-positive dendritic cells were not detected in normal mouse spinal cord.
   d: The number of CD11c-positive dendritic cells increases significantly by administration of GM-CSF.

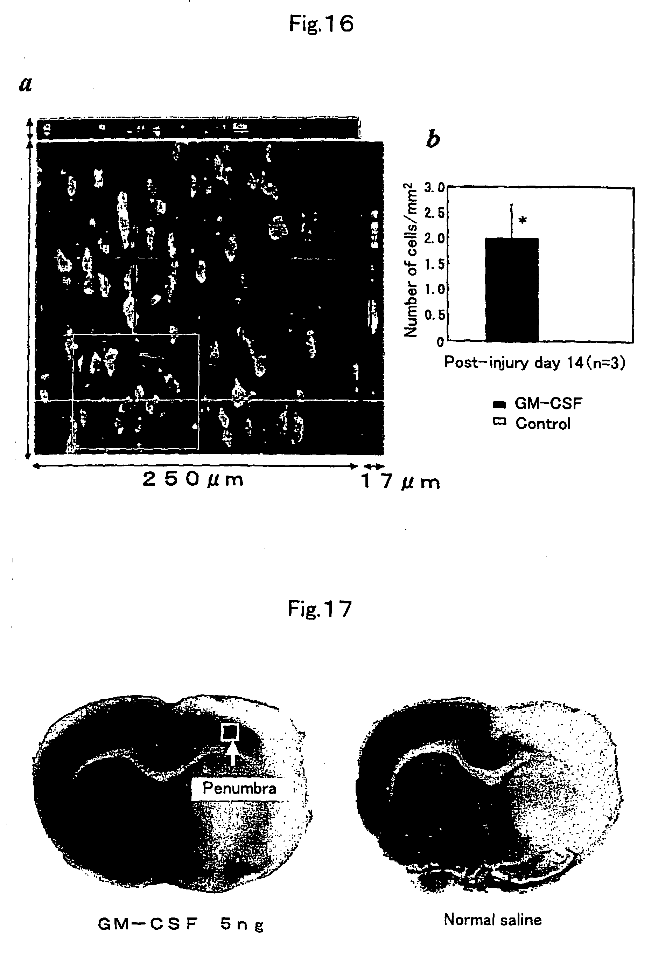

FIG. 16 shows the analysis results of nerve cells by administration of GM-CSF.
   a: A representative section with which analysis was performed of nerve cells newly formed, using anti-Hu antibody (green) and anti-BrdU antibody (red) on day 14 after injury.
   b: The number of both Hu-positive and BrdU-positive nerve cells newly formed on day 14 after injury.
FIG.17 shows a representative TTC staining 48 hours after cerebral infarction (MCAO).

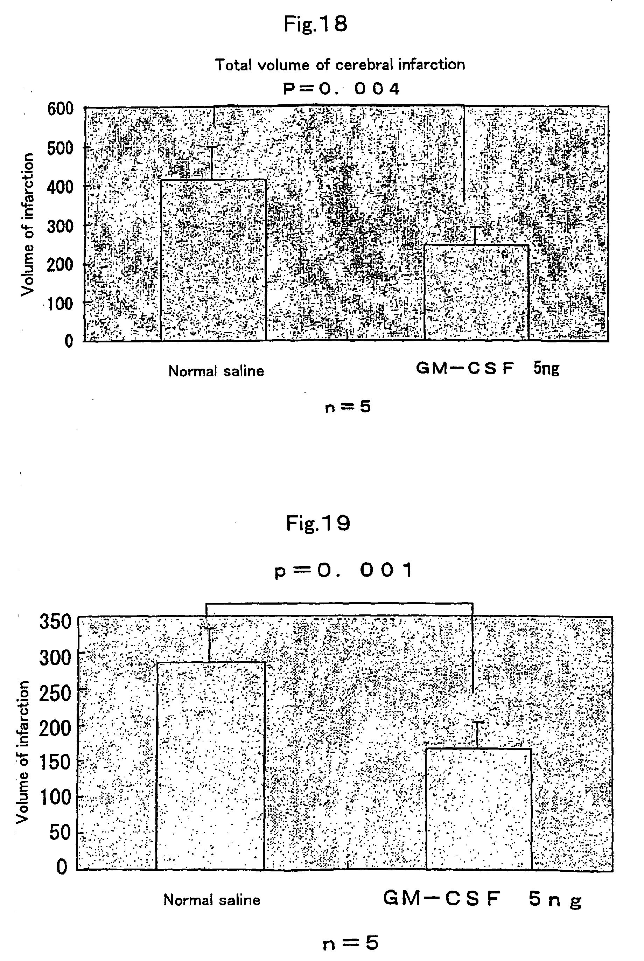

FIG. 18 shows that the total cerebral infarction volume decreases by intravascular administration of GM-CSF immediately after cerebral infarction.
FIG. 19 shows that the cerebral infarction volume in the cortex decreases by intravascular administration of GM-CSF immediately after cerebral infarction.

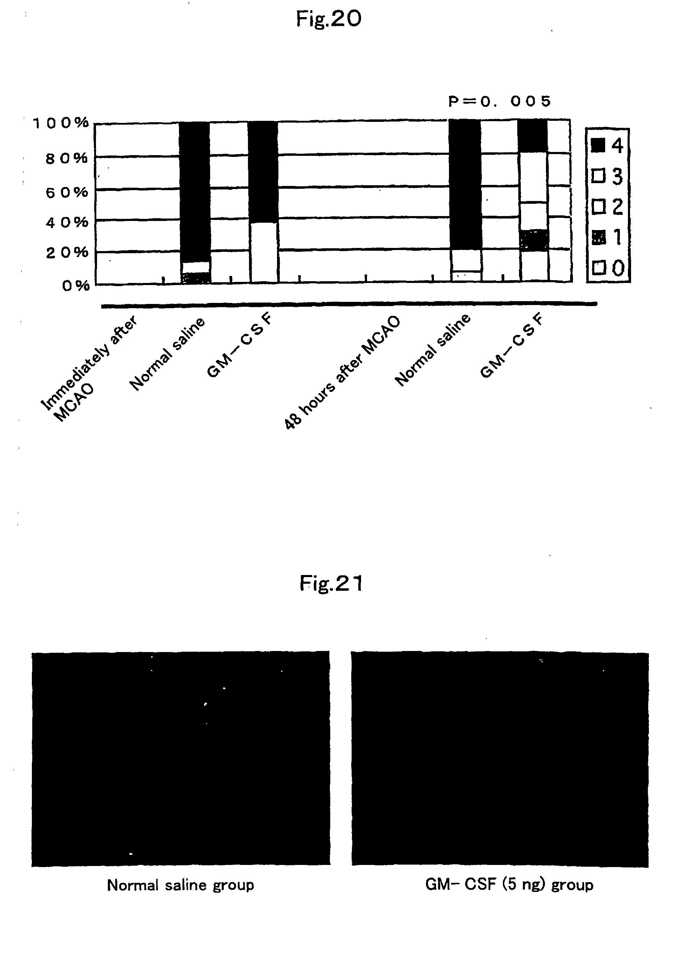

FIG. 20 shows neurological properties of the GM-CSF-administered group and the control group immediately and 48 hours after cerebral infarction.
FIG. 21 shows the results of analysis of apoptosis in the boundary region of cerebral infarction (penumbra) by TUNEL staining of the brain section prepared 48 hours after cerebral infarction (MCAO).

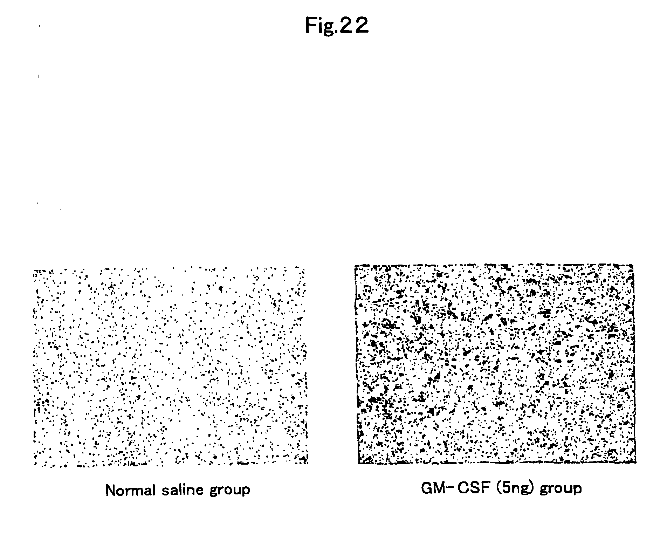

FIG. 22 shows the results of analysis of activated microglia in the boundary region of cerebral infarction (penumbra), using OX42 antibody to brain sections prepared 48 hours after cerebral infarction (MCAO).

### BEST MODE FOR CARRYING OUT THE INVENTION

The method for inducing proliferation of neural stem cells according to the present invention is not particularly limited, whether *in vitro*, *in vivo,* or *ex vivo,* as long as it is a method in which neural stem cells are contacted with at least one of dendritic cells, blood cells or culture supernatant of these cells, or granulocyte-macrophage colony stimulating factor (GM-CSF). In consideration of transplantation therapy requiring neural stem cells prepared in large quantities, however, a preferred method is to induce proliferation of neural stem cells, by contacting neural stem cells with at least one selected from a group consisting of dendritic cells, blood cells, and granulocyte-macrophage colony stimulating factor (GM-CSF) in a culture medium such as DMEM/F12 medium. Illustratively, a method for inducing proliferation of neural stem cells is to isolate and harvest mammalian nervous tissues (e.g., the putamen-corpus striatum of a fetus) containing the neural stem cells with the pluripotency to differentiate into three kinds of cells constituting the central nervous system, called neuron, astrocytes, and oligodendrocytes, together with a self-replication ability to divide and proliferate, selectively culture the neural stem cells in a culture medium containing growth factors, and subject the neural stem cells to a treatment for increasing the purity of the neural stem cells as necessary, followed by contacting the neural stem cells with dendritic cells and/or blood cells by co-culturing these cells in a culture medium. Another method for inducing proliferation of neural stem cells, for example, is to contact neural stem cells with GM-CSF by culturing them in the presence of GM-CSF, regardless of the presence or absence of co-culture with the above-mentioned dendritic cells and/or blood cells. Yet another preferred method for inducing proliferation of neural stem cells is illustratively to isolate nervous tissues containing the neural stem cells, selectively culture the neural stem cells in a culture medium containing growth factors like the above described, and then contact the neural stem cells by culturing them in the culture supernatant of dendritic cells and/or the culture supernatant of blood cells.

As the above-mentioned growth factors, epidermal growth factor (EGF), acidic fibroblast growth factor (aFGF or FGF-1), basic fibroblast growth factor (bFGF or FGF-2), transforming growth factor α (TGFα), amphiregulin, betacellulin (BTC), epiregulin (ER), heparin-binding EGF-like growth factor (HB-EGF), schwannoma-derived growth factor (SDGF), etc. can be illustratively shown; especially, EGF and FGF are illustratively preferred. Further, to a culture medium containing the above-mentioned growth factor(s), ingredients usually used for cell culture, such as transferrin, insulin, retinoic acid, activin, interleukin, etc. other than such growth factors as EGF and FGF, may be added.

Illustratively, the aforementioned dendritic cells are preferably an immature dendritic cell subset having the CD11c surface marker on the cell surface or a mature dendritic cell subset derived from the immature dendritic cell subset. Such dendritic cell subset includes a dendritic cell subset that secrete neurotrophic factor NT-3, which exerts *in vivo* nerve-regeneration effect, and induces proliferation and enhancement of phagocytosis of microglia; an immature dendritic cell subset that express, in addition to NT-3, CNTF (neurotrophic factor), which has the effect of protection from degeneration/cell death on both the motor and sensory nerves of the spinal cord, TGF-β1, which has the inhibitory effect on release of cytotoxic substances derived from microglia or macrophages, and IL-6, which induces the protection effect on various neurons (cholinergic, catecholaminergic, and dopaminergic neurons) ; and a mature dendritic cell subset that express EGF whose neuroprotective effect has been confirmed, CNTF, TGF-β1, and IL-6, in addition to NT-3. Alternatively, as the above-described mature dendritic cell subset, a mature dendritic cell subset obtained by culturing an immature dendritic cell subset *in vitro* in the presence of stimulants for maturing immature dendritic cells, such as LPS, IL-1, TNF-α, and CD 40L, may be used.

An immature dendritic cell subset having the CD11c surface marker on the cell surface can be obtained by isolating the dendritic cell subset by, for example, using a method of subjecting peripheral blood etc. to the pretreatment of density centrifugal separation treatment, etc., followed by sorting by FACS using a monoclonal antibody against a dendritic cell surface antigen or a separation method using a magnetic bead-coupled monoclonal antibody against dendritic cell surface antigen or the like, and then selecting the CD11c-positive dendritic cell subset out of the dendritic cell subset. Moreover, dendritic cells to be contacted with neural stem cells are preferably of the same kind as these neural stem cells. The preferred number of dendritic cells to be contacted with neural stem cells is 10³ or more of that of the neural stem cells in terms of the ratio of the number of cells because of marked induction of neural stem cell proliferation.

The aforementioned blood cells are illustratively spleen cells, T cells, monocytes, neutrophils, eosinophils, and basophils. Among these, T cells, especially CD8-positive T cells, and spleen cells are illustratively preferred.

Next, the set for inducing proliferation of neural stem cells according to the present invention is not particularly limited as long as it includes at least one kind of dendritic cells, blood cells or a culture supernatant of these cells, or granulocyte-macrophage colony stimulating factor (GM-CSF). However, preferred are the sets that further include a culture medium containing various ingredients such as growth factors including EGF and FGF. Further, the aforementioned dendritic cells are preferably an immature dendritic cell subset having the CD11c surface marker on the cell surface or a mature dendritic cell subset derived from the immature dendritic cell subset. The blood cells are preferably spleen cells, T cells, monocytes, neutrophils, eosinophils, or basophils. Treatment of illnesses, such as degenerative diseases, such as Parkinson's disease, Alzheimer's disease, and Huntington disease, traumatic and neurotoxic injuries to the central nervous system, and cerebral infarction resulting from the inhibition of blood flow or oxygen supply to the nervous system, etc. become possible by performing the method for inducing proliferation of neural stem cells according to the present invention in vitro or ex vivo and using the resulting neural stem cells, or performing the method for inducing proliferation of neural stem cells according to the present invention *in vivo.* Therefore, the neural stem cells or the sets for inducing proliferation thereof obtained by the present invention are useful as therapeutic agents for the above-mentioned nerve injuries or nerve function insufficiency. Further, administration of the neural stem cells obtained by the present method for inducing proliferation or the sets for inducing proliferation of neural stem cells according to the present invention enables treatment of the above-mentioned nerve injuries or nerve function insufficiency. For example, GM-CSF is useful as a therapeutic agent for spinal cord injuries or cerebral infarction; local or systemic administration of GM-CFS enables treatment of spinal cord injuries and cerebral infarction.

When using the set for inducing proliferation of neural stem cells as a therapeutic agent for nerve injuries or nerve function insufficiency, various mixing ingredients for preparation, such as a pharmaceutically acceptable common carrier, a binder, a stabilizer, an excipient, a diluent, a pH buffer, a disintegrator, a solubilizer, a solubilizing agent, and a tension agent, may be added to the aforementioned cancer therapeutic agent according to the present invention. Further, such a therapeutic agent may be administered orally or parenterally. That is, it may be administered orally in commonly used administration forms, for example, such as powder, granules, capsule, syrup, and suspension, or parenterally and locally in the form of an injection in dose forms such as a solution, an emulsion, and a suspension. The agent may also be administered in a spray form into the nostrils.

The present invention will be described in more detail by way of examples as follows, which should not be construed as limiting the technical scope of the present invention thereto.

The present invention will be more specifically described by giving examples hereinbelow. However, the scope of the present invention is not limited to these examples.

### EXAMPLE 1

### (ISOLATION OF DENDRITIC CELLS)

Immature dendritic cells were obtained by isolating a CD11c-positive subset from spleens of 6-week-old BALB/c or C57BL/6 female mice by the immunomagnetic bead method. Specifically, the spleens were first homogenated with 100 U/ml collagenase (Worthington Biochemical Corporation). Subsequently, the pellicle portion that was hard to separate was further incubated with 100 U/ ml collagenase for 20 min in 5% CO₂ at 37°C, and cells were separated. The cells obtained were suspended in 35% BSA solution, further overlaid with RPMI1640 + 10% fetus serum in a centrifuge tube, and then subjected to centrifugation at 3000 rpm for 30 min at 4°C. The cells at the interface layer between the 35% BSA solution and the RPMI1640 + 10% fetus serum solution were recovered. Next, by reacting for 15 min at 4°C magnetic bead-coupled monoclonal antibody (2 x 10⁸ beads, Miltenyi Biotech) against CD11c antigen with the recovered cells and magnetically separating the cells bound to the beads, the fraction in which the immature dendritic cell subset was concentrated was obtained.

### EXAMPLE 2

### (INDUCTION OF PROLIFERATION OF ENDOGENOUS NEURAL STEM CELLS/PRECURSORS BY TRANSPLANTATION OF DENDRITIC CELLS)

A laminectomy of the eighth thoracic vertebra was performed on six-week old BALB/c or C57BL/6 mice under ether anesthesia and by hemisecting the spinal cord on the left side with a scalpel, spinal cord-injured model mice were prepared. Immediately after injury, RPMI1640 medium with or without dendritic cells (1 x 10⁵ /mouse) obtained by sorting a CD11c (+) subset using the immunomagnetic bead method was transplanted into the injured site.

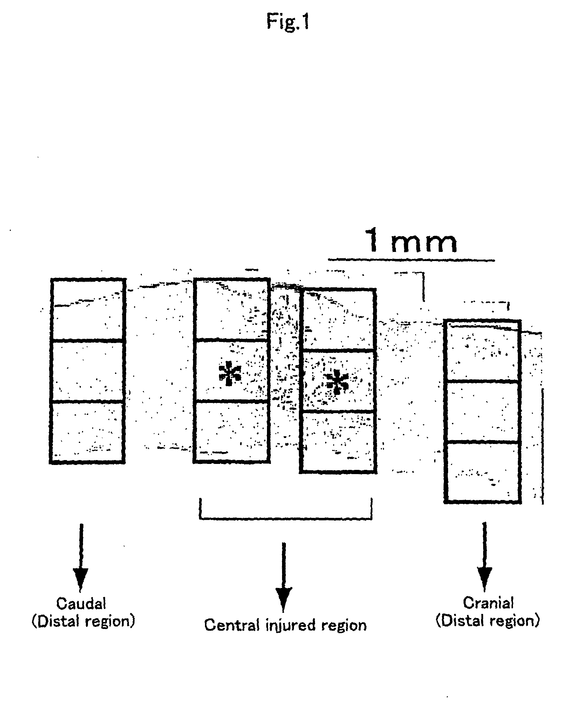

To examine the reactivity of endogenous neural stem cells/precursors by transplantation of dendritic cells, using Musashi-1 antibody, which recognizes the cells, immunohistochemical staining was performed to investigate time-course changes in the number of positive cells. First, dendritic cell-transplanted mice on day 2, 4, and 7 after injury were subjected to perfusion fixation with 2% paraformaldehyde through the heart and frozen sections were prepared (n=3/group). Next, immunohistological staining was performed using anti-mouse Musashi-1 antibody as the primary antibody. Musashi-1 is an RNA-binding protein with a molecular weight of about 38 kDa, identified by Okano et al. in 1994 (Neuron, 1994). It has been reported that Musashi-1 is strongly expressed in neural stem cells/precursors in the analysis using monoclonal antibody against mouse Musashi-1 (Dev. Biol. 1996, Neurosci. 1997, Dev. Neurosci. 2000). The measurement area was classified into two regions: the proximal and distal injured regions (cranial and caudal) as the regions from dorsal to ventral in each of the most distal region of the gelfoam (degenerative collagen), used when transplanting cells, and the point 1 mm away therefrom. (See FIG. 1).

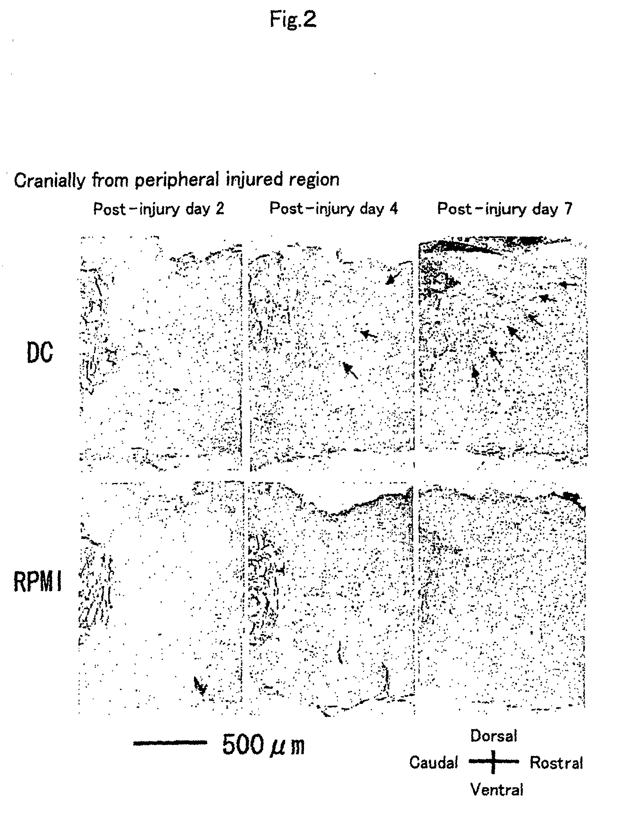

FIG.2 shows staining images of representative sections cranially from the proximal injured region. On day 2 after injury, no difference was observed in both groups, but on and after day 4 after injury a large number of Musashi-1-positive cells were observed in the dendritic cell-transplanted group. In contrast, almost no such changes were found in the control group.

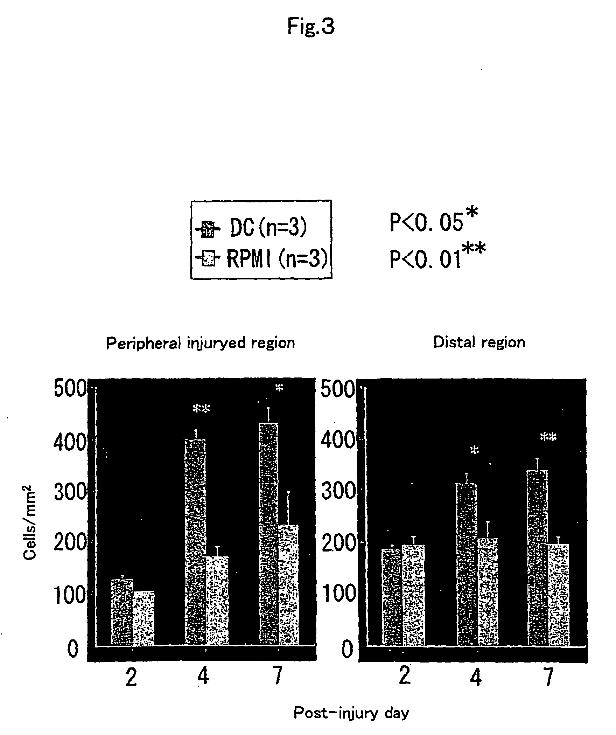

Next, Musashi-1-positive cells were quantitatively analyzed using an image analyzing device (Flovel Co., Ltd.). FIG.3 shows time-course changes in the number of Musashi-1-positive cells according to the area.

On and after day 4 after injury, a significant increase in the number of Musashi-1 positive cells due to transplantation of dendritic cells was observed both in the proximal and distal injured regions, as compared with the control. Especially, in the proximal injured region, a remarkable increase in the number of Musashi-1-positive cells was observed between day 2 and day 4 after injury in the dendritic cell-transplanted group.

In conclusion, it was revealed that transplantation of dendritic-cells to an injured site induces proliferation of endogenous neural stem cells/precursors.

### EXAMPLE 3

### (ANALYSIS of NERVE CELLS BY DENDRITIC CELL TRANSPLANTATION)

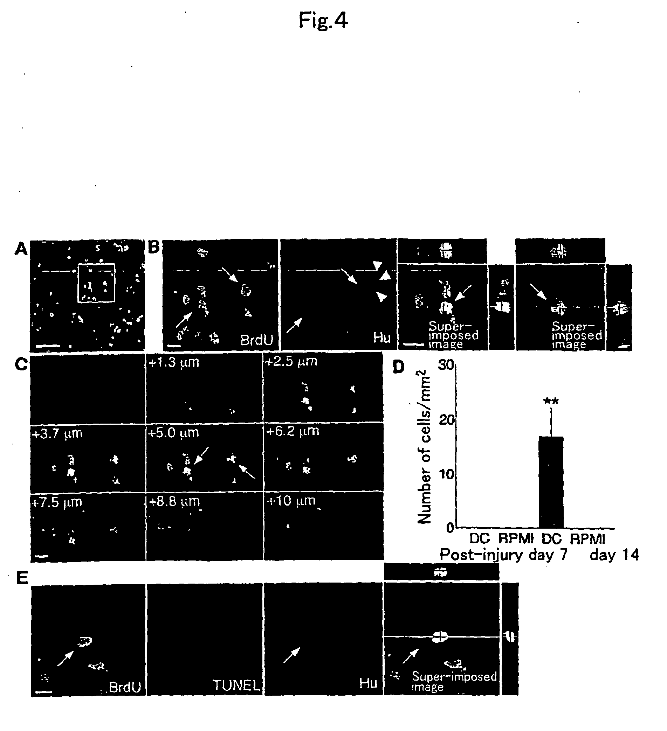

It was found that transplantation of dendritic cells significantly increased the number of endogenous neural stem cells/precursors. On day 14 after injury, however, the number of neural stem cells/precursors decreased as compared with day 7, and morphological changes were observed. Thus, on the assumption that neural stem cells might have differentiated into nerve cells, the possibility of de novo neurogenesis by transplantation of dendritic cells was examined. On days 7 and 14 after the spinal cord of mature C57BL/6 mice was injured and the dendritic cells were transplanted, the mice were subjected to perfusion fixation with 4% paraformaldehyde through the heart and sagittal frozen sections were prepared (n=3). The RPMI1640-transplanted group was used as the control (n=3). To label dividing, proliferating cells, the thymidine analogue bromodeoxyuridine (BrdU, Sigma Chemical Corporation) was administered intraperitoneally every day till the previous day of perfusion fixation (50mg/kg) after injury. Immunostaining was performed, using anti-rat BrdU antibody (Abcam Ltd.) and anti-Hu antibody (provided from Dr. James Okano) that recognizes neurons after mitosis as primary antibodies. The result of staining was confirmed using the confocal laser-scanning microscope (Carl Zeiss Inc.). The measurement was performed both cranially and caudally, making the measurement area all gray matter regions on the sections from the point 0. 5 mm away to the point 1 mm away from the most distal region of the gelfoam (degenerative collagen) that was used in transplantion of cells. FIG.4A shows a staining image of a representative section on day 14 after injury in the dendritic cell-transplanted group and the FIG. 4B and FIG. 4C show high-magnified images of the region shown by the square in FIG. 4A. The measurement results are shown in FIG. 4D. The results revealed that de novo neurogenesis occurred only in the dendritic cell-transplanted group on day 14 after injury (indicated by the arrow). In addition, since it is known that cells undergoing apoptosis also turn out BrdU-positive, the results of multiple immunostaining with the TUNEL method that enables specific detection of apoptotic cells are shown in FIG. 4E. The results revealed that transplantation of dendritic cells into the injured spinal cord had induced differentiation of new neurons because Hu/BrdU double positive cells were negative for TUNEL staining and fragmentation images characteristic of the nuclei of apoptotic cells were also negative. Among the central nervous system, the spinal cord has been considered a tissue in which de novo neurogenesis does not occur. The present invention, however, was able to demonstrate that in the spinal cord of mature mammalians, transplantation of dendritic cells results in de novo neurogenesis.

### EXAMPLE 4

### (REGENERATION OF INJURED NERVE AXONS BY DENDRITIC CELL TRANSPLANTATION)

To investigate the effect of transplantation of dendritic cells on cut nerve axons, using BALB/c mice after 4 months post-injury, the anterograde tracer biotinylated dextran amine (BDA, molecular weight 10000, Molecular Probes) was injected into the primary motor cortex of the cerebral cortex to examine regeneration of the tractus corticospinalis. The dendritic cell-transplanted mice on day 14 after injection of the tracer were subjected to perfusion fixation with 4% paraformaldehyde through the heart, and coronal frozen sections were prepared (n=10). The RPMI1640-transplanted group was used as control (n=7). In both groups, the BDA-positive nerve axons in the tractus corticospinalis were broken in the more cranial position than the gelfoam used in the cell transplantation and could not be detected in the more caudal region than the injured region (FIG. 5A). In the periphery of the central canal (marked with an asterisk) , in the more caudal region than the injured region, BDA-positive nerve axons (with a continuity of 0. 5 mm or more) were not observed in the control group (FIG.5B), whereas BDA-positive nerve axons were detected in the gray matter region that was not the tractus corticospinalislis in the dendritic cell-transplanted group (FIG. 5 C, n=5). A high-magnified image of that region is shown in FIG5D (marked with an arrow) and also in FIG.5E. The resulting nerve axons had a similar form (marked with arrows and arrowheads) in an example of regeneration of nerve axons reported in the past (Exp. Neurol. 1990, Science 1996, J. Neurosci. 2000). In conclusion, it was shown that transplantation of dendritic cells regenerates injured nerve axons.

### EXAMPLE 5

### (IN VITRO INDUCTION OF NEURAL STEM CELL PROLIFERATION BY CO-CULTURE WITH DENDRITIC CELLS)

Since it was found that transplantation of dendritic cells into an injured site induces proliferation of endogenous neural stem cells/precursors, it was analyzed whether or not dendritic cells can cause neural stem cell to proliferate in vitro as well. Induction of proliferation of neural stem cells was attempted by two-step isolation culture of neural stem cells. In the first step, the putamen-corpus striatum sites of C57BL/6 fetuses (on day 14 of gestation) were isolated and neural stem cells were cultured selectively by culturing the cells for 5 to 7 days at a cell density of 1 x 10⁵ cells/ml in a liquid medium composed of DMEM/F12 medium supplemented with 20 ng/ml EGF (PeproTech, Inc.), 20 ng/ml FGF-2 (R&D Systems), 100 µg/ml transferrin (Sigma Chemical Corporation), 25 µg /ml insulin (Sigma Chemical Corporation), 20 nM progesterone (Sigma Chemical Corporation), 30 nM sodium selenate (Sigma Chemical Corporation), and 60 µM putrescine (Sigma Chemical Corporation).

Further, to increase the purity of the neural stem cells obtained, PI staining-negative cells with a diameter of 10 µm or more were isolated using a cell sorter and plated at a density of 100 cells/well, and thus co-culture with dendritic cells was started. The cell sorter used was a Becton Dickenson FACS Vantege SE and for analysis Clone cyte plus was used. For the dendritic cells to be used for co-culture with neural stem cells, the CD11c-positive subset was isolated from the spleens of mature C57BL/ 6 female mice and prepared in the above-described liquid medium at a cell density of 1 x 10³ to 10⁵ cell/ml, and 100 µl of the cell suspension was added to each well of a 96-well low-adhesion culture plate. A group to which cells were not added (the fundamental condition for culture of neurospheres) and another group to which NT-3 (1 to 10 ng/ml), the most important neurotrophic factor secreted by dendritic cells, was added, were included as controls. The neural stem cells were thus cultured.

Since proliferated neural stem cells form cell aggregates, called neurosphere with a diameter of 50 µm or more, the number (FIG. 6) and the volume (FIG. 7) of neurospheres were measured and examined under individual conditions eight days after co-culture was started. The results revealed that neural stem cells had markedly proliferated due to co-culture with dendritic cells (DC), as compared with the conventional conditions for culture of neurospheres.

In the next step, the reactivity of neural stem cells associated with dendritic cells was examined, using spleen cells, T cells, and macrophages as controls. A total of 10⁵ (10⁶/ml) of each of these cells were co-cultured for seven days with 100 neural stem cells that had formed primary neurospheres. When the co-culture was performed with dendritic cells, the number of secondary neurospheres (with a diameter of 100 µm or more) formed remarkably increased as compared with the conventional culture in the neurosphere liquid medium alone (FIG. 8). In contrast, when co-culture was performed with T cells and macrophages, formation of secondary neurospheres with a diameter of 100 µm or more was not observed. These results revealed that dendritic cells, when co-cultured with neural stem cells *in vitro*, cause them to markedly proliferate, as compared with other immune cells.

### EXAMPLE 6

### (IN VITRO INDUCTION OF NEURAL STEM CELL PROLIFERATION USING DENDRITIC CELL CULTURE SUPERNATANT)

Further it was examined whether or not the substance secreted by dendritic cells can induce proliferation of neural stem cells. In this experiment, the number and volume of neurospheres were analyzed using the culture supernatants of not only dendritic cells but also spleen cells and T cells, which are blood cells. Neural stem cells were isolated using the same procedure as described in Example 4. That is, the putamen-corpus striatum sites of C57B L/6 fetuses (on day 14 of gestation) were isolated and neural stem cells were cultured selectively by culturing for 5 to 7 days at a cell density of 1 x 10⁵ cells/ ml in a liquid medium composed of DMEM/F12 medium supplemented with 20 ng/ml EGF (PeproTech, Inc.), 20 ng/ml FGF-2 (R&D Systems), 100 µg/ml transferrin (Sigma Chemical Corporation), 25 µg/ml insulin (Sigma Chemical Corporation), 20 nM progesterone (Sigma Chemical Corporation), 30 nM sodium selenate (Sigma Chemical Corporation), and 60 nM putrescine (Sigma Chemical Corporation). Further, to increase the purity of the neural stem cells obtained, after isolating PI staining-negative cells with a diameter of 10 µm or more using a cell sorter, the cells were plated at a density of 100 cells/ml.

Spleen cells were prepared from the spleens of mature C57BL/6 female mice. The CD11c-positive subset and the CD8-positive subset were isolated as dendritic cells and CD8T cells, respectively, and cultured in the aforementioned liquid medium for 24 hours. Subsequently, the culture supernatants were recovered. A group to which cells were not added (the fundamental condition for culture of neurospheres) was included as control and neural stem cells were cultured. Since proliferated neural stem cells form cell aggregates, called neurosphere with a diameter of 50 µm or more, eight days after co-culture was started, the number (FIG. 6) and volume (FIG.7) of neurospheres were measured and examined under individual conditions. The results revealed that neural stem cells had markedly proliferated due to the culture supernatants of not only dendritic cells (DCs) but also spleen cells (SPCs) and CD8-positive T cells (CD8-T), as compared with the conventional conditions under which neurospheres are cultured.

Next, to reconfirm that some substance in the culture supernatant secreted by dendritic cells causes neural stem cells to proliferate, 100 neural stem cells that had formed primary neurospheres were cultured for seven days using the culture supernatant of 10⁵ dendritic cells (10⁶ cells/ml, 48, 72 and 96 hours). Formation of secondary neurospheres (with a diameter of 50 µm or more) was observed (FIG. 11), though the number was about one-tenth of that found in the co-culture with 10⁵ dendritic cells. Since no formation of secondary neurospheres (with a diameter of 50 µm or more) was observed using the liquid medium alone, the presence of a secreted factor of dendritic cells that causes neural stem cells to proliferate was confirmed.

### EXAMPLE 7

### (INDUCTION OF PROLIFERATION OF ENDOGENOUS NEURAL STEM CELLS /PRECURSORS BY ADMINISTRATION OF GM-CSF)

To analyze the reactivity to neural stem cells/precursors in the central nervous system due to administration of GM-CSF, which is a cytokine important for induction and proliferation of dendritic cells, using Musashi-1 antibody, which recognizes these cells, immunohistological staining was performed for investigation of time-course changes in the number of positive cells. Spinal cord-injured model mice were prepared using six-week-old BALB/c female mice. Immediately after injury, 5 µl of normal saline with or without GM-CFS (250 pg/mouse, Genzyme Corporation) was administered to the injured site of the spinal cord (n=3/group). On days 2, 4, and 7 after injury, the mice were subjected to perfusion fixation with 2% paraformaldehyde through the heart and frozen sections were prepared. Next, immunohistological staining using anti-Musashi-1 antibody as the primary antibody was performed. As for the measurement area, the regions 0.5 mm dorsally and ventrally away from the most distal region of the gelfoam used in the cell transplantation, were quantitatively analyzed using an image analyzing device (Flovel Co., Ltd.) (FIG.12). Time-course changes in the number of Musashi-1 positive cells are shown in FIG.13. In the GM-CSF-administrated group, a large number of Musashi-1-positive cells were observed as early as day 2 after injury, as compared with the control, and a significant increase in the number of cells was observed on day 7. In conclusion, it was found that administration of GM-CSF to an injured site induces proliferation of endogenous neural stem cells/precursors within nervous tissue.

It was thus revealed that endogenous neural stem cells/precursors increased in number by administration of GM-CSF to the local site of injury. Further, analysis using the BrdU label to demonstrate that endogenous neural stem cells/precursors are proliferating cells and analysis using a glial cell marker (GFAP antibody) in addition to Musashi-1 antibody, which is a neural stem cells/precursor cell marker, were performed. Spinal cord-injured model mice were prepared using six-week-old mice and immediately after injury, 5 µl of normal saline alone or GM-CFS (250 pg/mouse, Genzyme Corporation) was administered to the injured site of their spinal cord. To label dividing, proliferating cells, the thymidine analogue BrdU (Sigma Chemical Corporation) was administered intraperitoneally (50 mg/kgm) every day after injury, till the previous day of perfusion fixation performed on post-injury day 7. Frozen sections were prepared and immunohistological analyses were performed using anti-rat BrdU antibody (Abcam Ltd.) and anti-mouse GFAP antibody (Sigma Chemical Corporation) as primary antibodies. After injury, the proliferating endogenous neural stem cells/precursors were measured as Musashi-1 (+), BrdU (+), and GFAP (-) cells. FIG.14a shows staining images of representative sections on day 7 after injury and FIG.14b further shows analytical images obtained by each antibody. The results of the quantitative measurement are shown in FIG.14c. It was confirmed that the number of endogenous neural stem cells/precursors significantly increased by administration of GM-CSF seven days after injury.

### EXAMPLE 8

### (INDUCTION OF INTRASPINAL DENDRITIC CELLS BY ADMINISTRATION OF GM-CSF TO AN INJURED SITE)

Spinal cord-injured model mice were prepared using six-week old mice and immediately after injury, 5 µl of normal saline alone or GM-CFS (250 pg/mouse, Genzyme Corporation) was administered to the injured site of their spinal cord. On day 7 after injury, immunohistological analyses were performed by subjecting the mice to perfusion fixation and preparing frozen sections. Dendritic cells in the spinal cord were analyzed using anti-CD11c antibody (BD Biosciences Pharmingen). Evident CD11c-positive dendritic cells were not detected in the normal mouse spinal cord (FIG.15c). However, CD11c-positive dendritic cells were detected (FIG.15b) in the injured spinal cord, where the number of dendritic cells further increased by administration of GM-CSF to an injured site (FIG.15a). A quantitative analysis was performed in the range of 0.75 to 1.25 mm cranially and caudally from the injured site. The number of CD11c positive dendritic cells significantly increased by administration of GM-CSF (GM-CSF, n=6; control, n=6; normal spinal cord mice, n=2) (FIG.15d). Dendritic cells transplanted to injured nerves induce proliferation of neural stem cells, exerting various neuroprotective and regenerative effects, such as de novo neurogenesis and regeneration of nerve axons. To date, it has been considered that dendritic cells are not present in the normal central nervous system. However, it has now been revealed that dendritic cells are induced to the central nervous system as well by injury and that much more dendritic cells are induced by administration of GM-CSF.

### EXAMPLE 9

### (ANALYSIS OF NERVE CELLS BY ADMINISTRATION OF GM-CSF)

Since it was found that transplantation of dendritic cells in the injured spinal cord induces differentiation of new neurons, analysis of nerve cells by administration of GM-CSF was performed. The spinal cord of mice was injured, subjected to perfusion fixation with 4% paraformaldehyde through the heart on day 14 after injury, and sagittal frozen sections were prepared (n=3). A normal saline-administered group was used as control (n=3). To label dividing, proliferating cells, the thymidine analogue BrdU (Sigma Chemical Corporation) was administered intraperitoneally every day till the previous day of perfusion fixation (50mg/kg, mouse) after injury. Immunostaining was performed using anti-rat BrdU antibody (Abcam Ltd.) and Hu antibody (provided by Dr. James Okano), which recognizes neurons after mitosis, as primary antibodies. The results of staining were confirmed using the confocal laser-scanning microscope (Carl Zeiss Inc.). The measurement was performed both cranially and caudally, making measurement area all gray matter regions on the sections from the point 0.25 mm away to the point 0.75 mm away from the most distal region of the gelfoam (degenerative collagen) used in the cell transplantation. FIG.16A shows a staining image of the representative section on day 14 after injury in the GM-CSF-administered group and FIG.16b shows the results of the quantitative measurement. The results revealed de novo neurogenesis only in the GM-CSF-administered group on day 14 after injury. In the central nervous system, the spinal cord has been considered a tissue in which de novo neurogenesis does not occur. However, the present invention was able to demonstrate that in the spinal cord of mature mammalians, administration of GM-CSF causes de novo neurogenesis.

### EXAMPLE 10

### (THERAPEUTIC EFFECT ON CEREBRAL INFARCTION AFTER ADMINISTRATION OF GM-CSF)

Next, the therapeutic effect by administration of GM-CSF on neurological disorders other than spinal cord injuries was examined. Cerebral infarction model rats were produced and analyses were performed on cerebral infarction lesions, neurological findings, apoptosis in the boundary region of cerebral infarction (penumbra), and activated microglia. As the cerebral infarction model, the transient middle cerebral artery occlusion (MCAO) model by the technique of Koizumi, Zea Longa, et al. was used Specifically, the following procedures were used. Male Wister rats were used. Anesthesia was introduced with premixed gas of nitrous oxide and oxygen (nitrous oxide: 70% and oxygen: 30%) together with 4% halothane and subsequently maintained at a lower concentration of halothane (2%). An operation was performed under spontaneous breathing. Amidline neck incision was made to expose the right common carotid artery, external carotid artery, internal carotid artery, and pterygopalatine A. After the internal carotid artery and common carotid artery were clamped with mini clips and pterygopalatine A. was ligated, the external carotid artery was separated and evaginated at the distal end. An embolus composed of a 4-0 nylon thread precoated with rubber (SURFLEX F, GC Corporation, Tokyo) was inserted from the external carotid artery and advanced for a distance of about 17 mm to occlude the origin of the middle cerebral artery. After 1 hour of ischemia, the embolus was removed. A polyethylene tube (PE 10, Intramedic, external diameter: 0.61 mm, internal diameter: 0.28 mm) was inserted from the external carotid artery and advanced to the internal carotid artery for injection of either 5 ng of GM-CSF /0. 3 ml of normal saline or 0. 3 ml of normal saline. After the tube was removed, the internal carotid artery and common carotid artery were released for reperfusion. The wound was closed and the operation was finished. For evaluation of the volume of cerebral infarction, the rats were sacrificed 48 hours after the reperfusion. Following perfusion with normal saline, the brains were then sectioned into 5 slices, each 2 mm in thickness. The sections were stained with a 2% solution of 2,3,5-trinphenyltetrazolium chloride (TTC) in normal saline (Sigma Chemical Corporation). (Stroke 1986 Nov-Dec; 17(6): 1304-8, Bederson JB, Pitts LH, Germano SM, Nishimura MC, Davis RL). Normal regions are stained red, whereas infarcted lesions are unstained and observed as white areas. TTC-staining images of each section were captured with a scanner. The area of infarction was calculated and the volume of infarction was obtained as the area of infarction multiplied by 2 mm. Further, total volume of cerebral infarction was obtained as the sum of volume of individual five slices for analysis. Neurological findings were analyzed using Menzies scale (Neurosurgery 1992Ju1; 31(1): 100-6; discussion 106-7; Menzies SA, Hoff JT, Betz AL). The scores of neurological observation by Menzies are as shown in Table 1.

**(Table 1)**

| Score | Evaluation |
|---|---|
| 0 | No apparent deficits |
| 1 | Contralateral forelimb flexion |
| 2 | Decreased grip of the contralateral forelimb while tail pulled |
| 3 | Spontaneous movement in all directions; contralateral circling only if pulled by tail |
| 4 | Spontaneous contralateral circling |

FIG.17 shows representative TTC-staining images 48 hours after cerebral infarction (MCAO). When GM-CSF was administered intravascularly immediately after cerebral infarction, an evident reduction in cerebral infarction lesions was observed. FIGS. 18 and 19 show the results of quantitative analyses. Evidently, administration of GM-CSF decreased not only the total volume of cerebral infarction but also the volume of cerebral infarction in the cerebral cortex. FIG. 20 shows neurological properties of the GM-CSF-administered group and the control group immediately and 48 hours after cerebral infarction. No evident difference was found between both groups immediately after cerebral infarction. In 48 hours, however, evidently, administration of GM-CSF significantly improved neurological properties obtained immediately after cerebral infarction.

To clarify what mechanism of action causes a decrease in cerebral infarction lesions, analysis was performed of cell death (apoptosis) in the boundary region of cerebral infarction (penumbra) (FIG.17 shows the boundary region of cerebral infarction after MCAO). Sections of the brain were made 48 hours after cerebral infarction (MCAO) and apoptosis was analyzed by TUNEL staining (Apotag kit; Intergen Co.). FIG. 21 shows representative TUNEL-staining images. Suppression of cell death by administration of GM-CSF was observed. Cell death in the boundary region of cerebral infarction was quantitatively analyzed and it was found that administration of GM-CSF evidently decreases cell death (the GM-CSF-administered group, n=7, 20.3 ± 6.0; the normal saline-administered group, and n=9; 73.2 ± 19.1/0.06 mm²: p<0.05; unpaired t-test)

Since it is known that GM-CSF activates microglia *in vitro* and that activated microglia secretes various neurotrophic factors, activated microglia were further analyzed in the boundary region of cerebral infarction by administration of GM-CSF. Brain sections were prepared 48 hours after cerebral infarction (MCAO), and microglia were analyzed using OX42 antibody (Serotec Inc.). FIG.22 shows representative OX42-staining images. It was found that administration of GM-CSF had increased the number of activated microglia. The number of cells was quantatively analyzed in the boundary region of cerebral infarction and it was observed that there was a tendency of increase of the number of activated microglial cells due to administration of GM-CSF (the GM-CSF-administered group, n= 8, 59.5 ± 16.5; the normal saline-administered group, n=9; 28.8 ± 5.1/0.24 mm²: p=0.08; unpaired t test).

In conclusion, it was considered that administration of GM-CSF activates microglia in the brain, whereby actions of secreted neurotrophic factors and the like have suppressed cell death.

### INDUSTRIAL APPLICABILITY

According to the present invention, it is possible to efficiently induce proliferation of neural stem cells *in vivo* and/or *in vitro,* which are the most important for transplantation therapy and so forth of nerve injuries or nerve function insufficiency.

## Claims

1. A method for inducing proliferation of a neural stem cell, comprising contacting the neural stem cell with at least one of a dendritic cell, a blood cell or the culture supernatant of the cell, or granulocyte-macrophage colony stimulating factor (GM-CSF).

2. The method for inducing proliferation of a neural stem cell of claim 1, comprising contacting the neural stem cell with at least one of a dendritic cell, a blood cell, and granulocyte-macrophage colony stimulating factor (GM-CSF) in a culture medium.

3. The method for inducing proliferation of a neural stem cell of claim 2, comprising isolating a mammalian nervous tissue containing the neural stem cell, selectively culturing the neural stem cell in a culture medium containing a growth factor, and then co-culturing the neural stem cell with a dendritic cell and/or a blood cell.

4. The method for inducing proliferation of a neural stem cell of claim 2, comprising isolating a nervous tissue containing the neural stem cell, selectively culturing the neural stem cell in a culture medium containing a growth factor, and then culturing the neural stem cell in the culture supernatant of at least one of a dendritic cell and a blood cell.

5. The method for inducing proliferation of a neural stem cell of any one of claims 2 to 4, wherein the culture medium containing the growth factor is a culture medium containing EGF and/or FGF.

6. The method for inducing proliferation of a neural stem cell of any one of claims 1 to 5, wherein the dendritic cell is an immature dendritic cell subset having the CD11c surface marker on the cell surface or a mature dendritic cell subset derived from the immature dendritic cell subset.

7. The method for inducing proliferation of a neural stem cell of any one of claims 1 to 6, wherein the blood cell is a spleen cell, a T cell, a monocyte, a neutrophil, an eosinophil, or an basophil.

8. A set for inducing proliferation of a neural stem cell, comprising at least one of a dendritic cell, a blood cell or the culture supernatant of the cell, or granulocyte-macrophage colony stimulating factor (GM-CSF).

9. The set for inducing proliferation of a neural stem cell of claim 8, further comprising a culture medium containing a growth factor.

10. The set for inducing proliferation of a neural stem cell of claim 9, wherein the culture medium containing the growth factor is a culture medium containing at least EGF and/or FGF.

11. The set for inducing proliferation of a neural stem cell of any one of claims 8 to 10, wherein the dendritic cell is an immature dendritic cell subset having the CD11c surface marker on the cell surface or a mature dendritic cell subset derived from the immature dendritic cell subset.

12. The set for inducing proliferation of a neural stem cell of any one of claims 8 to 11, wherein the blood cell is a spleen cell, a T cell, a monocyte, a neutrophil, an eosinophil, or a basophil.

13. A therapeutic agent for a nerve injury or nerve function insufficiency, comprising containing as an active ingredient the neural stem cell obtained by the method for inducing proliferation of any one of claims 1 to 7.

14. A therapeutic agent for a nerve injury or nerve function insufficiency, comprising containing as an active ingredient the set for inducing proliferation of any one of claims 8 to 12.

15. ,. A therapeutic agent for cerebral infarction, comprising containing granulocyte-macrophage colony-stimulating factor (GM-CSF) as an active ingredient.

16. A therapeutic method for a nerve injury or nerve function insufficiency, comprising administering the neural stem cell obtained by the method for inducing proliferation of any one of claims 1 to 7.

17. A therapeutic method for a nerve injury or nerve function insufficiency, comprising administering the set for inducing proliferation of any one of claims 8 to 12.

18. A therapeutic method for cerebral infarction, comprising administering granulocyte-macrophage colony-stimulating factor (GM-CSF).
